# EUROPEAN PATENT SPECIFICATION

(11) **EP 1 713 771 B1**
(45) Date of publication and mention of the grant of the patent: **05.03.2014**
(21) Application number: 05700893.0
(22) Date of filing: 13.01.2005
(51) Int. Cl.: C07D 209/42, A61K 31/475, A61P 9/12

(54) **PROCESS FOR THE PREPARATION OF A NEW CRYSTALLINE FORM OF PERINDOPRIL**
VERFAHREN ZUR HERSTELLUNG VON EINER NEUEN KRISTALLINEN FORM VON PERINDOPRIL
PROCEDE POUR LA PREPARATION D'UNE NOUVELLE FORME CRISTALLINE DE PERINDOPRIL

(30) Priority: 14.01.2004 SI 200400012
(43) Date of publication of application: 25.10.2006
(73) Proprietor: LEK Pharmaceuticals d.d., 1526 Ljubljana (SI)
(72) Inventor: RUCMAN, Rudolf, 1211 Ljubljana-Smartno (SI)
(86) International application number: PCT/EP2005/000283
(87) International publication number: WO 2005/068425

(56) References cited:
- EP-A- 0 308 341
- EP-A- 1 296 948
- EP-A- 1 371 659
- WO-A-03/064388
- DE-A1- 19 721 290
- VINCENT M ET AL: "SYNTHESIS AND ACE INHIBITORY ACTIVITY OF THE STEREOISOMERS OF PERINDOPRIL (S 9490) AND PERINDOPRILATE (S 9780)" DRUG DESIGN AND DISCOVERY, HARWOOD ACADEMIC PUBLISHERS GMBH, vol. 9, no. 1, 1992, pages 11-28, XP000885876 ISSN: 1055-9612

## Description

### Field of the invention

The present invention relates to a process for the preparation of a new crystalline form of perindopril.

### Background of the invention

Perindopril with the following structural formula I: 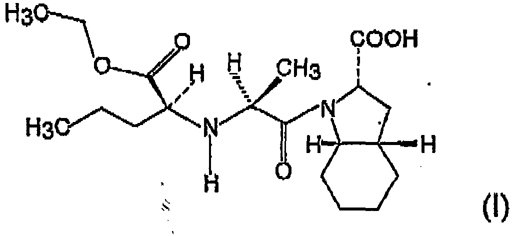 is (2S, 3aS, 7aS)-1[(2S)-2-[[(1S)-1-ethoxycarbonyl)butyl]amino]propionyl] octahydro-1H-indole-2-carboxylic acid. Perindopril acts as a transport form ("prodrug") of the diacid perindoprilat which is its active form. After oral administration perindopril is rapidly absorbed in the body and primarily metabolized in the liver, to perindoprilat and its inactive metabolites. It is an ACE Inhibitor used in the treatment of cardiovascular diseases, especially In the treatment of hypertension and heart failure.

ACE inhibitors ("Angiotensin Converting Enzyme") inhibit the conversion of angiotensin I to angiotensin II. They are antihypertensive agents also acting as vasodilators.

Perindopril was first disclosed in EP 0049658 B1 and US 4,508,729. The preparation process described therein is rather complex and leads to a mixture of stereoisomers, which must be separated. N,N'-dicyclohexylcarbodiimide is used as condensation reagent. In this patent perindopril as a pure (S) isomer and its sodium salt are also disclosed.

DE 197 21 290 describes a process for the preparation of ACE inhibitors, wherein substituted alanine derivatives are silylated and activated with thionylchloride to give silylated acid chloride derivatives which are reacted with appropriate amino acids with protected carboxylic groups. The corresponding ACE inhibitors are obtained by removal of the protecting groups.

WO 03/064388 describes a process for perindopril synthesis wherein N-[(S)-carbethoxy-1-butyl]-(S)-alanine is activated with thionylchloride to form the corresponding acid chloride of N-[(S)-carbethoxy-1-butyl]-(S)-alanine with a protected NH group intermediate, which is further reacted with (2S, 3aS, 7aS)-octahydroindole-2-carboxylic acid. The acid chloride is formed by the use of thionylchloride at ambient or slightly higher temperature. The NH group is protected by an N-alkoxy carbonyl group, e.g. N-ethoxycarbonyl-, N-methoxycarbonyl- and N-benzyloxycarbonyl group. The shortcoming of the synthesis is, on the one hand, the poor stability of N-alkoxycarbonyl groups in strongly acid chlorination conditions, and on the other hand the excessive stability of the N-benzyloxycarbonyl group which requires catalytic hydrogenation to be removed.

Perindopril obtained according to prior art processes is a colourless or slightly yellowish viscous oily substance which solidified in cold. Perindopril has been not isolated in crystalline form yet There have been disclosed only crystalline forms of perindopril addition salt - perindopnl erbumine by EP 1296947 B1, EP 1294689 A1 and EP 1296948 B1.

### Summary of the Invention

In one embodiment the present invention relates to a process for the preparation of the new crystalline form of perindopril comprising the steps of dissolving amorphous perindopril in a lower dialkyl ether containing up to 8 carbon atoms, and crystallizing said new crystalline form of perindopril from the resulting solution, wherein said new crystalline form is characterized by an X-ray powder diffraction pattern comprising characteristic peaks at (2θ): 5.101, 8.615, 9.373, 11.090, 11.403_{,} 15.230, 15.990, 17.215, 19.011, 19.506, 20.124 and 20.568;
and wherein
said amorphous perindopril is obtained by the process comprising the following steps:
(i) the secondary amino group of the stereospecific amino acid N-[(S)-carbethoxy-1-butyl]-(S)-alanine of formula II: 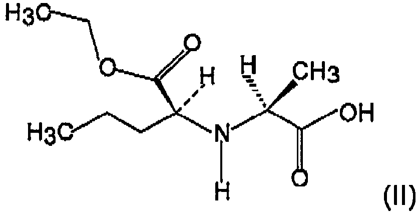 is protected with trimethylsilyl groups In order to obtain the compound of formula III: 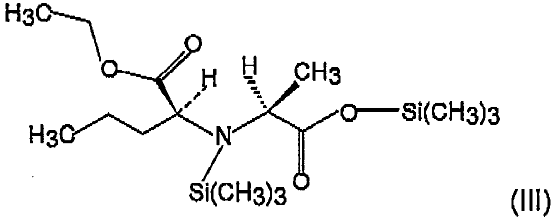
(II) compound (III) is then activated by reaction with an activating agent selected from the group consisting of thionylchloride and the complex of thionylchloride with 1-H-benzotriazole to obtain the acid chloride of formula IV: 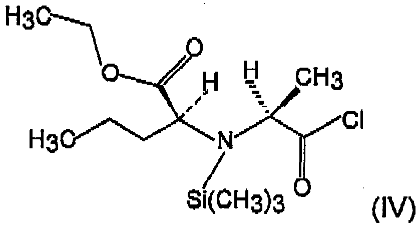
(iii) the acid chloride of formula IV is reacted with the (2S, 3aS, 7aS)-octahydroindolo-2-carboxylic acid having a protected carboxylic group of formula V: 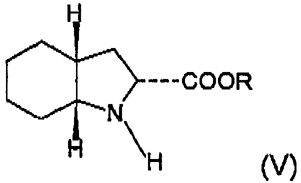 wherein R is a
   trimethylsilyl group, to obtain a protected perindopril derivative of formula VI: 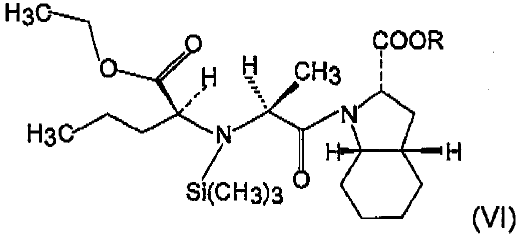
(Iv) the derivative of formula VI is then converted to perindopril of formula I by removal of trimethylsliyl groups during the step of removing impurities by extraction with an aqueous solution,
(v) the resulting organic phase containing perindopril is dried by evaporation of the solvent in vacuum,
(vi) the crude perindopril is dissolved in methylene chloride and filtered through a silica gel layer,
(vii) the fraction containing perindopril is evaporated under a strong vacuum or
said amorphous perindopril is obtained by the process comprising the following steps:
(i) the secondary amino group of the stereospecific amino acid N-[(S)-carbethoxy-1-butyl]-(S)-alanine of formula II: 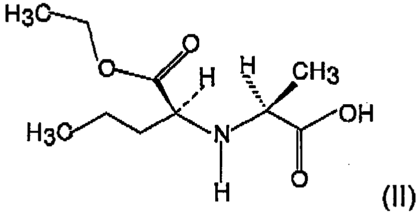 Is protected with trimethylsilyl groups in order to obtain the compound of formula III: 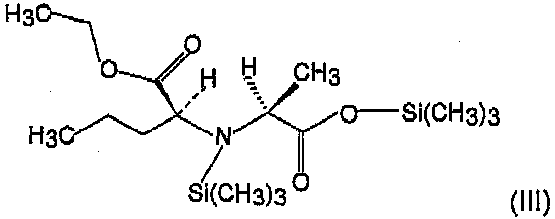
(II) compound (III) is then activated by reaction with an activating agent selected from the group consisting of thionylchloride and the complex of thionylchloride with 1- H-benzotriazole to obtain the acid chloride of formula IV: 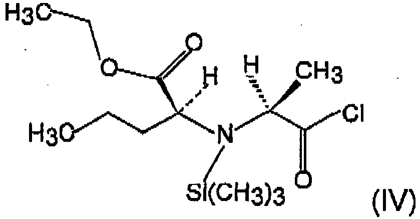
(iii) the acid chloride of formula IV is reacted with the (2S, 3aS, 7aS)-octehydroindolo-2-carboxylic acid having a protected carboxylic group of formula V: 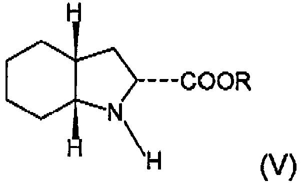 wherein R is a
   benzylic group, to obtain a protected perindopril derivative of formula VI: 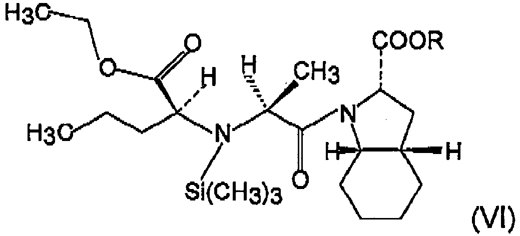
(iv) impurities from the final reaction mixture are removed by extraction with an aqueous solution,
(v) the organic phase is filtered through an aluminium oxide layer,
(vi) the fraction containing the derivative of formula VI is evaporated under vacuum,
(vii) the derivative of formula VI is then converted to perindopril of formula I by removal of all protecting groups by catalytic hydrogenation,
(viii) the catalyst is removed by filtration,
(ix) the solvent is evaporated under a vacuum.

### Description of the Figures

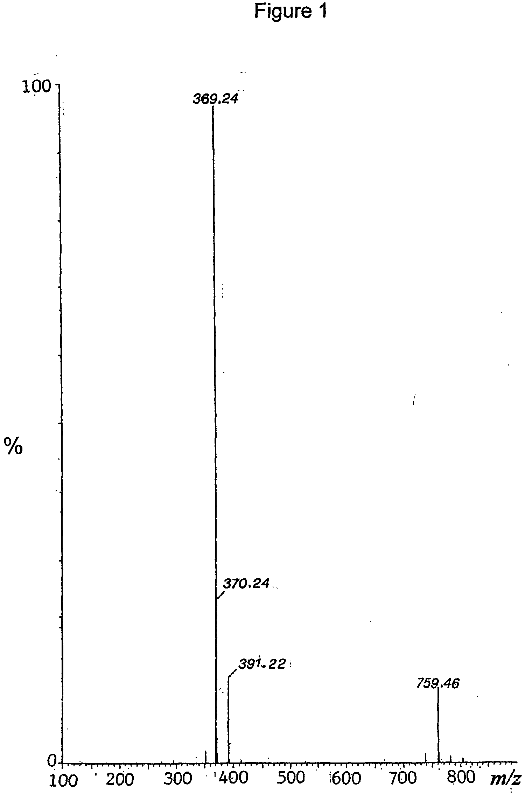

Figure 1. Mass spectrum of the new crystalline form of perindopril (recorded using the TOF MS ES+ technique).

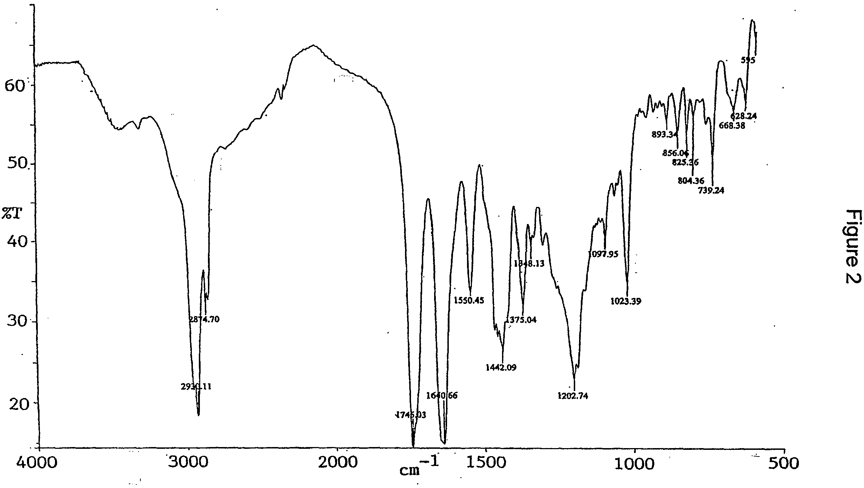

Figure 2. IR spectrum of the new crystalline form of perindopril.
Figure 3. X-ray diffraction diagram of the new crystalline form of perindopril.
Figure 4. NMR spectrium of the new crystalline form of perindopril.

### Detailed description of the Invention

Disclosed is a process for the preparation of perindopril of formula I 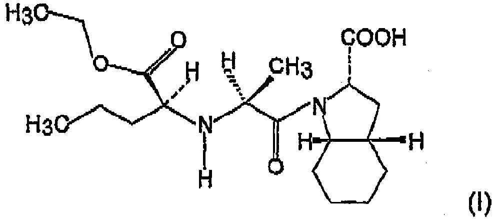 comprising the following steps:
(i) the secondary amino group of the stereospecific amino acid N-[(S)- carbethoxy-1-butyl]-(S)-alanine of formula II: 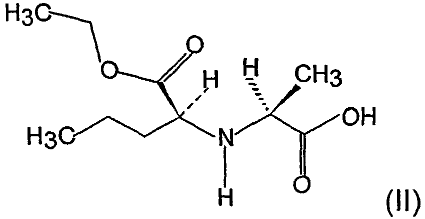 is protected with trimethylsilyl groups in order to obtain the compound of formula III: 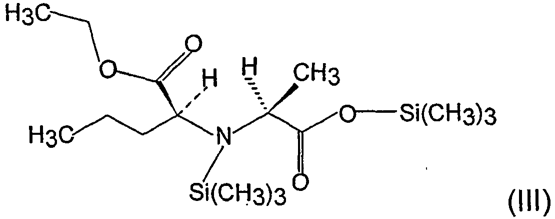
(ii) compound (III) is then activated by reaction with an activating agent selected from the group consisting of thionylchloride and the complex of thionylchloride with 1- H-benzotriazole to obtain the acid chloride of formula IV: 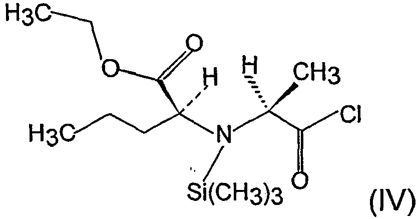
(iii) the acid chloride of formula IV is reacted with the (2S, 3aS, 7aS)-octahydroindolo-2-carboxylic acid having a protected carboxylic group of formula V: 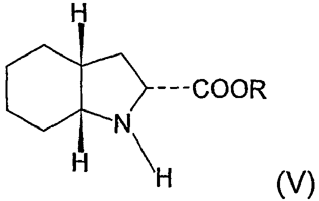 wherein R is a
   trimethylsilyl, tertiary butylic or benzylic group, to obtain a protected perindopril derivative of formula VI: 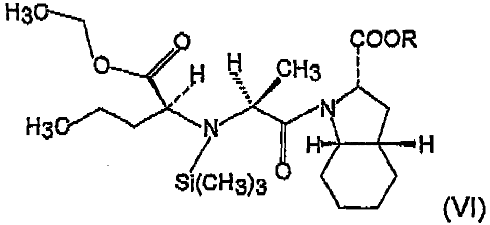
(iv) the derivative of formula VI Is then converted to perindopril of formula I by removal of all protecting groups.

Advantages of the process for the preparation of perindopril include the use of entirely protected reactants and the use of a very reactive reagent for the activation of such a form, such that the reaction can be carried out under mild conditions. The formation of disintegration products and of epimers is very low, Consequently, the yields are also adequately high. All raw materials and reagents are commercially available and their industrial production is possible.

In a preferred variant, the process may comprise a further step of isolating perindopril, whereby impurities are removed from the final reaction mixture by extraction with an aqueous solution and the resulting organic phase containing perindopril is dried by evaporation of the solvent in vacuum.

It is well known In the field of the peptide chemistry (G. A. Grant, Synthetic Peptides, W. H. Freeman and Co. New York, 1992, p. 81) that in the coupling of two amino acids all the remaining side reactive groups must be protected with adequate protective groups which prevent side reactions. At the end of the synthesis the protective groups must be removable without harm to the new compound. Protection of side groups is particularly important in the case of activation of carboxylic group with thionylchloride which is extremely reactive. Consequently, when thionylchloride is used as activating agent in step (ii) of the process the reaction should be carried out at low temperatures, ideally at temperatures below -10°C.

The alternative to using thionylchloride as the activating agent is to employ the reactive thionylchloride complex with 1-H-benzotriazole, which is an excellent reagent for the preparation of acid or alkaline chlorides in mild reactive conditions as the reaction is not exothermic. A reagent in the ratio of both components 1: 1 (S.S. Chaudari et al, Synlett 1999, 11, 1763) may also be prepared in advance in the form of solution in methylene chloride, because it is stable and does not show thionylchloride aggressiveness. The side reactions, particularly chlorination on the α-position regarding to carbonyl group, are essentially diminished.

The deprotection of protected perindopril of formula VI in step (iv) may be performed by different methods depending on the type of protecting group.

The trimethylsilyl protecting groups may be removed by water hydrolysis. For instance, the trimethylsilyl protecting groups can be removed in contact with water during the phase of perindopril isolation after completion of the synthesis.

The tert-butylic and benzylic groups need to be removed in a dedicated step, e.g. by use of strong acids or by catalytic hydrogenation. It is important to remove all traces of sulphur from the crude product comprising protected perindopril derivative of formula VI before catalytic hydrogenation in order to avoid inactivation of the catalyst. For this purpose a filtration step through a suitable layer is sufficient. Active basic aluminium oxide is one example of a suitable layer. The preferred activity of such a layer is 2-3. An appropriate thickness of such a layer is preferably about 2-4cm, e.g. 3 cm.

Unexpectedly, we have found that very pure perindopril can be obtained by filtration of the end-product of step (iv) of the process as already described. The filtration step may be performed by dissolving the crude substance in methylene chloride and passing it through a silica gel layer, e.g. Merck 60, granulation 0.2-0.0063. Silica gel may be wetted by a mixture of methylene chloride/ethanol 94/6 (vol/vol) and eluated with the same mixture. The resulting perindopril product is in the form of a viscous oil, which is converted into a colourless, amorphous solid substance by evaporation of traces of residual solvents under a strong vacuum. Amorphous chemical substances are characterized by the lack of diffraction peaks on a powder X-ray diffractogram.

Furthermore, we have found that amorphous perindopril can alternatively be obtained by the process by performing additional step of filtering the protected perindopril derivative of formula VI through a layer of active basic aluminium oxide before performing catalytic hydrogenation.

This is the first disclosure of the existence or isolation of amorphous perindopril. The amorphous perindopril according to the present invention shows no diffraction peaks in a powder X-ray diffraction diagram.

Solid amorphous perindopril may be converted to the addition salt with tert-butylamine by crystallization In a suitable solvent, e. g. methylene chloride, ether, tert-butyl methyl ether, ethyl acetate, butyl acetate, 2-pentanone, cyclolhexane, methyl cyclohexane, or similar solvents.

We have surprisingly found that amorphous perindopril, when dissolved In lower dialkyl ether containing up to 8 carbon atoms (e.g. diethyl ether), crystallizes In the form of colourless crystals that melt at 110-114°C. This new crystalline form of perindopril has been characterized by IR, MS, NMR and X-ray spectra. The new crystalline form of perindopril is highly water-soluble; 20 g is dissolved In 100 ml of water at 20°C. It is not hygroscopic and is therefore suited to incorporation into pharmaceutical compositions.

Also disclosed is a process for the preparation of the amorphous form of perindopril comprising the additional steps of:
- (v) filtration of crude perindopril obtained in step (iv) of the process according to the present invention through a silica gel layer, and
- (vi) evaporation of traces of any residual solvents under vacuum.

Also disclosed is a process for the preparation of the amorphous form of perindopril of formula I comprising the following steps:
(i) the secondary amino group of the stereospecific amino acid N-[(S)- carbethoxy-1-butyl]-(S)-alanine of formula II is protected with trimethylsilyl groups In order to obtain the compound of formula III,
(ii) compound (III) is then activated by reaction with an activating agent selected from the group consisting of thionylchloride and the complex of thionylchloride with 1- H-benzotriazole to obtain the acid chloride of formula IV,
(iii) the acid chloride of formula IV is reacted with the (2S, 3aS, 7aS)-octahydroindolo-2-carboxylic acid having a protected carboxylic group of formula V, wherein R is a trimethylsilyl group, to obtain a protected perindopril derivative of formula VI,
(iv) the derivative of formula VI Is then converted to Perindopril of formula I by removal of trimethylsilyl groups during the step of removing impurities by extraction with an aqueous solution,
(v) the resulting organic phase containing perindopril is dried by evaporation of the solvent in vacuum,
(vi) the crude perindopril is dissolved in methylene chloride and filtered through a allica gel layer,
(vii) the fraction containing perindopril is evaporated under a strong vacuum.

Also disclosed is a process for the preparation of the amorphous form of perindopril of formula I comprising the following steps:
(I) the secondary amino group of the stereospecific amino acid N-[(S)- carbethoxy-1-butyl]-(S)-alanine of formula II is protected with trimethylsilyl groups In order to obtain the compound of formula III,
(ii) compound (iii) is then activated by reaction with an activating agent selected from the group consisting of thionylchloride and the complex of thionylchloride with 1- H-benzotriazole to obtain the acid chloride of formula IV,
(iii) the acid chloride of formula IV is reacted with the (2S, 3aS, 7aS)-octahydroindolo-2-carboxylic acid having a protected carboxylic group of formula V, wherein R is a benzylic group, to obtain a protected perindopril derivative of formula VI,
(iv) impurities from the final reaction mixture are removed by extraction with an aqueous solution,
(v) the organic phase is filtered through an aluminium oxide layer,
(vi) the fraction containing the derivative of formula VI is evaporated under vacuum,
(vii) the derivative of formula VI is then converted to perindopril of formula I by removal of all protecting groups by catalytic hydrogenation, .
(viii) the catalyst is removed by filtration,
(ix) the solvent is evaporated under a vacuum.

An embodiment of the present invention is a process for the preparation of a new crystalline form of perindopril characterized in that amorphous perindopril obtained according to any of the above disclosed processes is dissolved In a lower dialkyl ether (e.g. diethyl ether) and then is allowed to crystallize. Preferably the crystallization is carried out at a temperature in the range 0 to 35° C, more preferably 5 to 25° C. Best results are obtained if the crystallization is carried out in two stages - firstly at a higher temperature (e.g. 15-30° C, more preferably 20-25° C) and subsequently at a lower temperature, such as 0 to 10° C, preferably 2-5° C). For instance, one may carry out the first stage of crystallization for 3 hours at room temperature and then for 6 hours at +5°C.

Also disclosed are pharmaceutical compositions that may on the one hand comprise amorphous perindopril or a salt or derivative thereof in combination with one or more pharmaceutically acceptable carriers or excipients. Alternatively, the pharmaceutical compositions may comprise perindopril in the new crystalline form disclosed herein or a salt or derivative thereof, (preferably the tert-butyl amine salt), in combination with one or more pharmaceutically acceptable carriers or exciplents.

Pharmaceutically acceptable excipients may be selected from the group consisting of binders, diluents, disintegrating agents, stabilizing agents, preservatives, lubricants, fragrances, flavoring agents, sweeteners and other excipients known In the field of the pharmaceutical technology. Preferably, carriers and excipients may be selected from the group consisting of hydroxypropylcellulose, lactose, microcrystalline cellulose, calcium carbonate, starch, colloidal silicone dioxide, sodium starch glycolate, talc, magnesium stearate, polyvinylpyrrolidone.

Optionally, the pharmaceutical compositions may be combination products comprising one or more additional pharmaceutically active components in addition to perindopril. Preferably, an additional pharmaceutically active component is a diuretic, e.g. indapamide.

Suitable pharmaceutical compositions are solid dosage forms, such as tablets with immediate release or sustained release of the active principle, effervescent tablets or dispersion tablets and capsules,

The pharmaceutical compositions may be prepared by methods known In the field of the pharmaceutical technology.

A therapeutically effective amount of perindopril Is an amount, which is appropriate In a dosage form useful to treat cardiovascular diseases, e.g. hypertension and heart failure. In general, from a pharmaceutically effective amount is 1 to 15 mg of perindopril, preferably 2 to 8 mg.

The present invention is illustrated by the following examples:

### Examples

### Example 1

### Preparation of perindopril erbumine

Stage I: Preparation of silylated (2S, 3aS, 7aS)-octahydroindole-2-carboxylic acid To a suspension of 2.0 g (0.0077 mole) (2S, 3aS, 7aS)-octahydroindole-2-carboxylic acid in 24 ml of methylene chloride at room temperature are added 0.98 ml of trimethylchlorosilane (0.0077 mole) and 1.08 ml (0.0077 mole) of triethylamine under stirring. The mixture is stirred at room temperature for 75 minutes.

### Stage II: Preparation of silylated N-[(S)-carbethoxy-1-butyl]-(S)-alanine

To a solution of 1.68 g (0.0077 mole) of N-[(S)-carbethoxy-1-butyl]-(S)-alanine in 30 ml of methylene chloride, cooled to -15°C are added, under stirring 1.96 ml (0.0155 mole) of trimethylchlorosilane and 2.15 ml (0.00155 mole) of triethylamine. The solution is allowed to rest at -15°C for 2.5 hours and stirred from time to time.

### Stage III: Preparation of perindopril

Thionylchloride complex with 1-H-benzotriazole (1:1), prepared from 0.92 g of 1-H-benzotriazole (0.0077 mole) and 0.56 ml of thionylchloride (0.0077 mole), diluted with 5 ml of methylene chloride, is added to the solution obtained in Stage II under stirring. The mixture is further stirred for 30 minutes.

To this solution the silylated (2S, 3aS, 7aS)- octahydroindole-2-carboxylic acid, prepared in Stage I, is added under stirring. The obtained solution is first stirred while the solution is heated from -15°C to +20°C, and then stirring is continued for additional 24 hours. The reaction may be followed using the analytical HPLC method, as described in Example 5.

When the reaction is complete, 30 ml of water is added, the pH value of the suspension is adjusted to 4.38 ±0.1 with 6 M NaOH (2.7 ml). After that 8 g of NaCl is added and stirred well. Organic and aqueous layers are separated. The methylene chloride phase is washed with 10% NaCl solution. The combined NaCl solutions with pH 4.4 are extracted twice with 15 ml of methylene chloride. The combined methylene chloride extracts are dried with MgSO₄ and evaporated in a vacuum at 40-50°C. 5.10 g of yellow dry matter comprising perindopril are obtained.

### Stage IV: Purification of perindopril

Dry matter obtained in Stage III is dissolved in methylene chloride (12 ml) and applied to a column filled with 50 g of silica gel (Merck 60, granulation 0.2-0.0063 mm), which is wetted with a mixture of methylene chloride/ethanol 94/6 (vol./vol.). 1-H-benzotriazole and perindopril are washed from the column separately with this solvent.

The process of separation is followed by the TLC method, as described in Example 5. 2.48 g (87.5%) of solid amorphous substance is obtained after evaporation of perindopril solution.

### Stage V: Preparation of perindopril erbumine

Amorphous perindopril obtained in Stage IV is dissolved in a mixture of methyl cyclohexane/ethanol (20 ml + 2 ml) at 70°C and decolourised with 0.5 g of activated charcoal. To the solution 0.68 ml of tert-butylamine in 3 ml of methyl cyclohexane and 1 ml of ethanol is added. Half of the solvent is evaporated in vacuum at 70°C. After crystallisation at -15°C overnight 2.0 g of perindopril erbumine (72.6 % of the theoretical yield) is obtained. Purity according to HPLC is approximately 99-99.5% (area). Melting temperature is 124-135°C.

### Example 2

### Preparation of perindopril erbumine

### Stage I: Preparation of silylated N-[(S)-carbethoxy-1-butyl]-(S)-alanine

1.88 g (0.00866 mole) of N-[(S)-carbethoxy-1-butyl]-(S)-alanine is suspended in 30 ml of methylene chloride. The solution is cooled to -15°C, and then 2.2 ml of trimethylchlorosilane (0.0173 mole) and 2.40 ml of triethylamine (0.0173 mole) is added under stirring. The solution is allowed to rest at -15°C for 90 minutes.

To this solution is added under stirring 0.63 ml (0.00866 mole) of thionylchloride in 6 ml of methylene chloride. The solution is allowed to rest at -15°C and stirred periodically.

### Stage II: Preparation and purification of perindopril

To the solution obtained in Stage I 2.24 g (0.00866 mole) of (2S, 3aS, 7aS)-octahydroindole-2-carboxylic acid benzyl ester in 10 ml of methylene chloride is added under stirring. The solution is allowed to rest at -15°C for 22 hours, then 2 ml of triethyl amine is added under stirring, and stirring is continued for a further 11 hours at 20°C. The reaction mixture is extracted with 25 ml of 9 %NaCl, 2x25 ml of saturated NaHCO₃, and dried with MgSO₄. The obtained solution is filtered through a layer (3 cm) of aluminium oxide (basic, activity 2-3). The solvent is evaporated in vacuum (3.13 g, oil).

The obtained oil is dissolved in methanol (50 ml) and 1.0 g of palladium on active charcoal (10%) is added, followed by hydrogenation with oxygen at 35-40°C for 8 hours. The catalyst is filtered off, washed with methanol and the solvent is evaporated in a vacuum at 40-50°C. 2.32 g (72.7 % yield) of dry white substance is obtained.

### Stage III: Preparation of perindopril erbumine

Perindopril obtained in Stage II is crystallized from 2-pentanone (30 ml) after the addition of 0.7 ml of tert-butylamine in 3 ml of 2-pentanone. The solution is allowed to rest for 1-2 hours at room temperature and overnight at -15°C. 2.33 g (83.8 %) of white crystals with melting point 120-130°C was obtained.

### Example 3

### Preparation of perindopril erbumine

Stage I: Preparation of silylated (2S, 3aS, 7aS)-octahydroindole-2-carboxylic acid 2.92 g of (2S, 3aS, 7aS)-octahydroindole-2-carboxylic acid perchlorate (0.00812 mole corresponding to 2.10 g of free acid) is suspended in 26 ml of methylene chloride. To the solution 1.03 ml (0.00812 mole) of trimethylchlorosilane and 2.25 ml (0.01624 mole) of triethylamine are added. The mixture is stirred at 20° C for 2 hours.

### Stage II: Preparation of silylated N-[(S)-carbethoxy-1-butyl]-(S)-alanine

1.76 g (0.00812 mole) of N-[(S)-carbethoxy-1-butyl]- (S)-alanine is suspended in 26 ml of methylene chloride and cooled to -15°C. To this solution. 2.05 ml (0.01624 mole) of trimethylchlorosilane and 2.25 ml (0.01624 mole) of triethylamine are added under stirring. The solution is allowed to rest at -15°C for 5 minutes and stirred from time to time.

### Stage III: Preparation of perindopril

To the solution obtained in Stage II 0.59 ml (0.00812 mole) of thionylchloride in 5 ml of methylene chloride is added. The solution is allowed to rest at -15°C for 45 minutes and stirred from time to time. Then a solution of silylated (2S, 3aS, 7aS)-octahydroindole-2-carboxylic acid, prepared in Stage I, is added under stirring. The resulting solution is allowed to rest overnight (for 15 hours) at -15°C. Then 2 ml of triethylamine is added and the mixture is stirred at room temperature for a further 14 hours.

To the reaction mixture 25 ml of water is added and the pH is adjusted to 4.1 ± 0.1 by the addition of a solution of 6M NaOH. 3 g of NaCl is dissolved in the aqueous phase, followed by strong stirring. The resulting phases are separated and the methylene chloride phase is extracted twice more with 25 ml of 9% NaCl water solution. The pH of the combined NaCl solutions is adjusted to 4.4 ± 0.1, and then the NaCl solution is re-extracted 3 times with 20 ml of methylene chloride. The resulting methylene chloride extracts are washed with 30 ml of 9% NaCl solution and then combined with the primary methylene chloride fraction. The methylene chloride fraction is dried with MgSO₄, and evaporated to dryness in vacuum at 50°C. 2.49 g of crude perindopril is obtained (80%, purity approx. 96%, area).

### Stage IV: Preparation of perindopril erbumine

Perindopril obtained in Stage III is dissolved in ethyl acetate (25 ml) at 60-70°C. To the solution 0.72 ml of tert-butylamine and 1 g of activated charcoal are added. The solution is finely filtered and perindopril erbumine is crystallized at -15°C overnight. 2.38 g of crystals (79.7 %) with melting point 118-130°C are obtained.

### Example 4

### Preparation of crystalline perindopril

### Stage I: Preparation of perindopril

The synthesis of perindopril is performed by the process described in Example 3, using the following reagents:
a) For the preparation of silylated (2S, 3aS, 7aS)-oct.3.hydroindole-2-carboxylic acid 3.80 g of (2S, 3aS, 7aS)-oct.3.hydroindole-2-carboxylic acid (0.01467 mole) in 45 ml of methylene chloride, 1.86 ml (0.01467 mole) of trimethylchlorosilane, and 2.04 ml (0.01467 mole) of triethylamine are used.
b) For the preparation of silylated N-[(S)-carbethoxy-1-butyl]-(S)-alanine: 3.18 g (0.01467 mole) of N-[(S)-carbethoxy-1-butyl]-(S)-alanine, 3.71 ml (0.02934 mole) of trimethylchlorosilane and 4.07 ml (0.02934 mole) of triethylamine are used.
c) For activation of silylated N-[(S)-carbethoxy-l-butyl]-(S)-alanine 1.07 ml (0.01467 mole) of thionylchloride is used.

5.77 g of solid crude perindopril is obtained according to the process described in Example 3.

### Stage II: Purification of perindopril

Crude perindopril obtained in Stage I is dissolved in 15 ml of methylene chloride and filtered through a column prepared with 30 g of silica gel (Merck 60, granulation 0.2-0.063 mm), wetted in a mixture of methylene chloride/ethanol 94/6 (vol./vol.). The eluate comprising perindopril is evaporated in a vacuum at 50°C to obtain 4.09 g of perindopril (75.9 %).

### Stage III: Preparation of crystalline perindopril

Perindopril obtained in Stage II is dissolved in ethyl ether and crystallized first at room temperature for 3 hours and then at +5°C for 5 hours. 3.20 g of perindopril in the new crystalline form are obtained (78.2% of the theory amount). Melting temperature is 110-114°C.

The solution is characterized by IR, NMR, MS spectra.

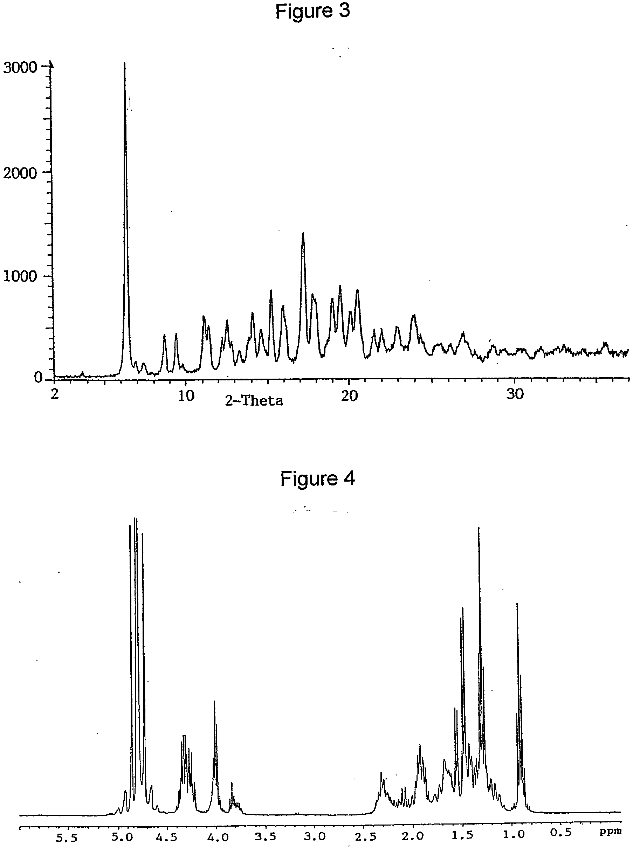

The powder X-ray diffraction diagram of the new crystalline form of perindopril was recorded by Siemens D 5000 diffractometer using the reflection technique under the following conditions: CuK_{α} radiation, range between 2° and 37°, step 0.04°, integration time: 1 second. The diffraction diagram (Figure 3) illustrates the intensities and relative intensities expressed in terms of angle 2θ or distance d:

| angle 2θ | inter-planar distance d ×10⁻¹⁰ m | intensity impulses. sec⁻¹ | relatative intensity % |
|---|---|---|---|
| 3.656 | 24.15027 | 72.0 | 2.4 |
| 5.101 | 17.31096 | 35.0 | 1.2 |
| 6.378 | 13.84687 | 3042 | 100 |
| 6.884 | 12.83052 | 165 | 5.4 |
| 7.362 | 11.99804 | 154 | 5.1 |
| 7.842 | 11.26490 | 67.0 | 2.2 |
| 8.615 | 10.25496 | 434 | 14.3 |
| 9.373 | 9.42781 | 444 | 14.6 |
| 9.719 | 9.09325 | 141 | 4.6 |
| 11.090 | 7.97163 | 612 | 20.1 |
| 11.403 | 7.75386 | 514 | 16.9 |
| 12.240 | 7.22540 | 403 | 13.2 |
| 12.524 | 7.06220 | 567 | 18.6 |
| 12.840 | 6.88883 | 354 | 11.6 |
| 13.294 | 6.65438 | 271 | 8.9 |
| 13.800 | 6.41169 | 400 | 13.1 |
| 14.082 | 6.28392 | 636 | 20.9 |
| 14.572 | 6.07381 | 477 | 15.7 |
| 15.230 | 5.81266 | 848 | 27.9 |
| 15.990 | 5.53829 | 700 | 23.0 |
| 17.215 | 5.14680 | 1403 | 46.1 |
| 17.958 | 4.93545 | 764 | 25.1 |
| 18.640 | 4.75633 | 328 | 10.8 |
| 19.011 | 4.66440 | 772 | 25.4 |
| 19.506 | 4.54719 | 891 | 29.3 |
| 20.124 | 4.40874 | 643 | 21.1 |
| 20.568 | 4.31453 | 850 | 27.9 |
| 21.572 | 4.11599 | 466 | 15.3 |
| 22.025 | 4.03235 | 472 | 15.5 |
| 22.983 | 3.86650 | 492 | 16.2 |
| 23.933 | 3.71499 | 607 | 20.0 |
| 23.347 | 3.65278 | 422 | 13.9 |
| 25.321 | 3.5.1446 | 328 | 10.8 |
| 25.520 | 3.48752 | 319 | 10.5 |
| 26.105 | 3.41063 | 327 | 10.7 |
| 29.885 | 3.31348 | 447 | 14.7 |
| 27.612 | 3.22783 | 266 | 8.7 |
| 28.560 | 3.12283 | 268 | 8.8 |
| 28.756 | 3.10197 | 309 | 10.2 |
| 29.428 | 3.03266 | 267 | 8.8 |
| 30.303 | 2.94702 | 277 | 9.1 |
| 30.593 | 2.91983 | 280 | 9.2 |
| 31.413 | 2.84537 | 261 | 8.6 |

### Example 5

System to follow the reaction process (HPLC):
Column: HD-Sil (ORPEGEN) 18-5s-100, 5 µm, 250 x 4.6 mm
Mobile phase: A) 25 % acetonitrile/75 % buffer pH=2 B) 70 % acetonitrile/30 % buffer pH=2.

Buffer composition: 0.92 g of sodium heptanesulfonate, 1 ml of triethylamine in 1000 ml of solution, pH 2.0 adjusted with perchloric acid.
gradient: 100% A 1 min, then to 100 % B in 25 min
flow rate: 1.25 ml/min
detection: UV at 200 nm.

System for the control of filtration on silica gel or Al₂0₃ column (TLC):
plates: silica gel, Merck 60;
mobile phase: methylene chloride/ethanol 90/10 (vol./vol.)
detection: Dragendorff's reagent.

Rf values:
perindopril 0.44
1-H-benzotriazole 0.66
triethylamine HCl 0.20.

### Example 6

Characterization of the new crystalline form of perindopril and the new amorphous form of perindopril

MS spectrum is obtained by Auto Spec Micromass spectrometer, TOF MS ES⁺ technique.

Infrared spectrum is obtained with Bio-Rad FTS-60 spectrophotometer. Samples are analyzed in KBr. -

¹H-NMR spectra is obtained with Varian NMR, 300 MHz, solvent D₂O, external standard TMSPO=0.

### Example 7

Composition of a mixture for preparing 1000 tablets containing 4 mg of active ingredient in each tablet:

| | |
|---|---|
| perindopril from example 4 | 3.34 g |
| tert-butylamine | 0.66 g |
| hydroxypropyl cellulose | 2 g |
| starch | 10 g |
| lactose | 100 g |
| magnesium stearate | 3 g |
| talc | 3 g |

Tert-butylamine is dissolved in the solvent appropriate for preparing a granulate, crystalline perindopril obtained in Example 4 is added and stirring is continued until perindopril is dissolved. The rest of the ingredients are added under stirring and the mixture is granulated.

## Claims

1. A process for the preparation of the new crystalline form of perindopril of formula I: 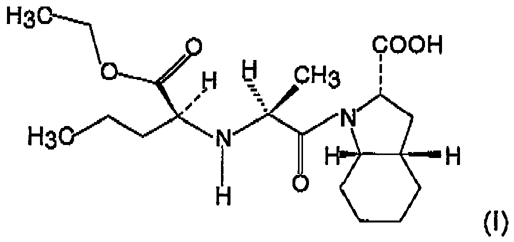 comprising the steps of dissolving amorphous perindopril in a lower dialkyl ether containing up to 8 carbon atoms, and crystallizing said new crystalline form of perindopril from the resulting solution, wherein said new crystalline form is **characterized by** an X-ray powder diffraction pattern comprising characteristic peaks at (2θ): 5.101, 8.615, 9.373, 11.080, 11.403, 15.230, 15.990, 17.215, 19.011, 19.506, 20.124 and 20.568;
and wherein
said amorphous perindopril is obtained by the process comprising the following steps:
(i) the secondary amino group of the stereospecific amino acid N-[(s)-carbethoxy-1-butyl]-(S)-alanine of formula II: 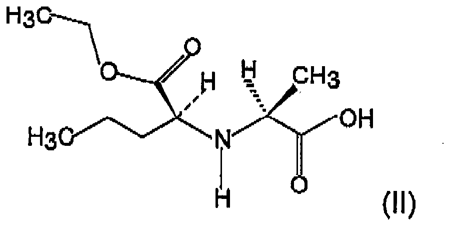 is protected with trimethylsilyl groups in order to obtain the compound of formula III: 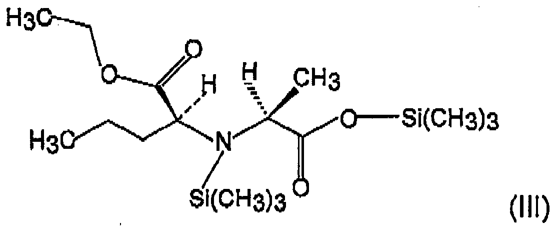
(ii) compound (III) is then activated by reaction with an activating agent selected from the group consisting of thionylchloride and the complex of thionylchloride with 1- H-benzotriazole to obtain the acid chloride of formula IV: 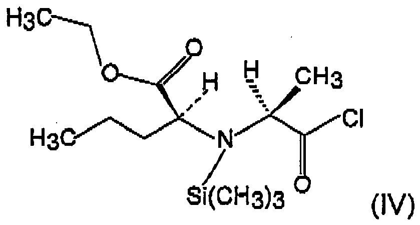
(III) the acid chloride of formula IV is reacted with the (2S, 3aS, 7aS)-octahydroindolo-2-carboxylic acid having a protected carboxylic group of formula V: 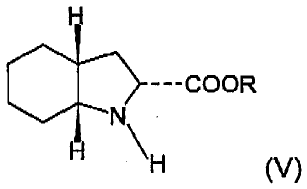 wherein R is a
trimethylsilyl group, to obtain a protected perindopril derivative of formula VI: 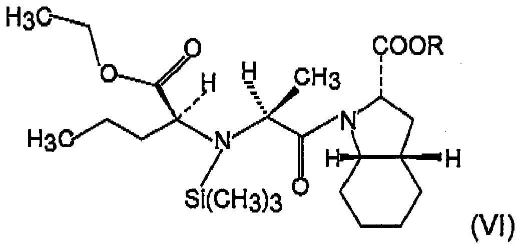
(iv) the derivative of formula VI is then converted to perindopril of formula I by removal of trimethylsilyl groups during the step of removing impurities by extraction with an aqueous solution,
(v) the resulting organic phase containing perindopril is dried by evaporation of the solvent In vacuum,
(vl) the crude perindopril is dissolved in methylene chloride and filtered through a silica gel layer,
(vii) the fraction containing perindopril is evaporated under a strong vacuum or
said amorphous perindopril Is obtained by the process comprising the following steps:
(i) the secondary amino group of the stereospecific amino acid N-[(S)-carbethoxy-1-butyi]-(S)-alanine of formula II: 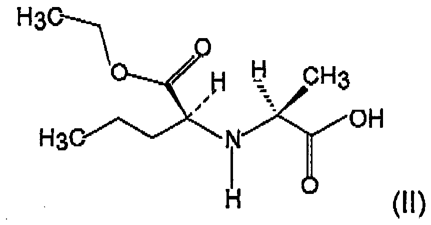 is protected with trimethylsilyl groups in order to obtain the compound of formula III: 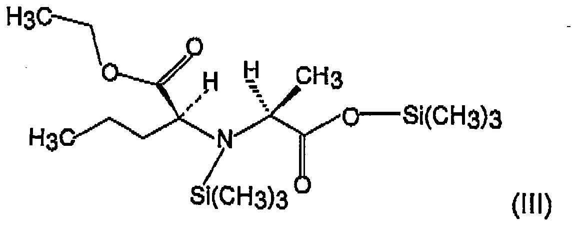
(ii) compound (III) is then activated by reaction with an activating agent selected from the group consisting of thionylchloride and the complex of thionylchloride with 1- H-benzotriazole to obtain the acid chloride of formula IV: 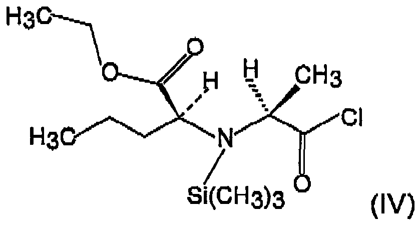
(iii) the acid chloride of formula IV Is reacted with the (2S, 3aS, 7aS)-octahydroindolo-2-carboxylic acid having a protected carboxylic group of formula V: 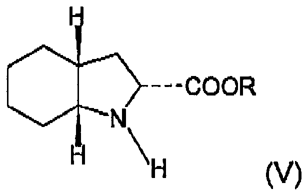 wherein R is a
benzylic group, to obtain a protected perindopril derivative of formula VI: 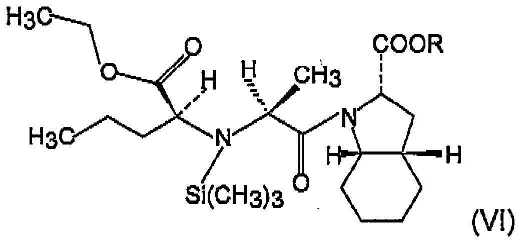
(iv) impurities from the final reaction mixture are removed by extraction with an aqueous solution,
(v) the organic phase is filtered through an aluminium oxide layer,
(vi) the fraction containing the derivative of formula VI is evaporated under vacuum,
(vii) the derivative of formula VI is then converted to perindopril of formula I by removal of all protecting groups by catalytic hydrogenation,
(viii) the catalyst is removed by filtration,
(ix) the solvent is evaporated under a vacuum.

2. A process according to claim 1, wherein said new crystalline form is **characterized by** the following powder X-ray diffraction diagram, measured using a diffractometer (CUK_{α} anticathode), expressed in terms of units of angle 2θ, with the inter-planar distance d and the corresponding intensity or relative intensity:
| angle 2θ | inter-planar distance d ×10⁻¹⁰ m | intensity impulses. sec⁻¹ | relatative intensity % |
|---|---|---|---|
| 3.656 | 24.15027 | 72.0 | 2.4 |
| 5.101 | 17.31096 | 35.0 | 1.2 |
| 6.378 | 13.84687 | 3042 | 100 |
| 6.884 | 12.83052 | 165 | 5.4 |
| 7.362 | 11.99804 | 154 | 5.1 |
| 7.842 | 11.26490 | 67.0 | 2.2 |
| 8.615 | 10.25496 | 434 | 14.3 |
| 9.373 | 9.42781 | 444 | 14.6 |
| 9.719 | 9.09325 | 141 | 4.6 |
| 11.090 | 7.97163 | 612 | 20.1 |
| 11.403 | 7.75386 | 514 | 16.9 |
| 12.240 | 7.22540 | 403 | 13.2 |
| 12.524 | 7.06220 | 567 | 18.6 |
| 12.840 | 6.88883 | 354 | 11.6 |
| 13.294 | 6.65438 | 271 | 8.9 |
| 13.800 | 6.41169 | 400 | 13.1 |
| 14.082 | 6.28392 | 636 | 20.9 |
| 14.572 | 6.07381 | 477 | 15.7 |
| 15.230 | 5.81266 | 848 | 27.9 |
| 15.990 | 5.53829 | 700 | 23.0 |
| 17.215 | 5.14680 | 1403 | 46.1 |
| 17.958 | 4.93545 | 764 | 25.1 |
| 18.640 | 4.75633 | 328 | 10.8 |
| 19.011 | 4.66440 | 772 | 25.4 |
| 19.506 | 4.54719 | 891 | 29.3 |
| 20.124 | 4.40874 | 643 | 21.1 |
| 20.568 | 4.31453 | 850 | 27.9 |
| 21.572 | 4.11599 | 466 | 15.3 |
| 22.025 | 4.03235 | 472 | 15.5 |
| 22.983 | 3.86650 | 492 | 16.2 |
| 23.933 | 3.71499 | 607 | 20.0 |
| 23.347 | 3.65278 | 422 | 13.9 |
| 25.321 | 3.51446 | 328 | 10.8 |
| 25.520 | 3.48752 | 319 | 10.5 |
| 26.105 | 3.41063 | 327 | 10.7 |
| 29.885 | 3.31348 | 447 | 14.7 |
| 27.612 | 3.22783 | 266 | 8.7 |
| 28.560 | 3.12283 | 268 | 8.8 |
| 28.756 | 3.10197 | 309 | 10.2 |
| 29.428 | 3.03266 | 267 | 8.8 |
| 30.303 | 2.94702 | 277 | 9.1 |
| 30.593 | 2.91983 | 280 | 9.2 |
| 31.413 | 2.84537 | 281 | 8.6 |

3. A process for the preparation of the new crystalline form of perindopril according to claim 1 or claim 2, wherein said lower dialkyl ether is diethyl ether.

## Patentansprüche

1. Verfahren zur Herstellung einer neuen kristallinen Form von Perindopril der Formel I: 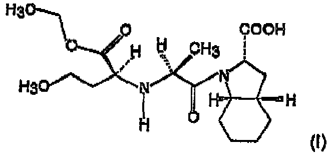 umfassend die Schritt des Auflösens von amorphem Perindopril in einem niederen Dialkylether enthaltend bis zu 8 Kohlenstoffatomen, und des Kristallisierens dieser neuen kristallinen Form von Perindopril aus der resultierenden Lösung, wobei die neue kristalline Form durch ein Röntgenpulverdiffraktionsmuster gekennzeichnet ist, welches charakteristische Peaks bei (2θ):5,101, 8,615, 9,373, 11,090, 11,403, 15,230, 15,990, 17,215, 19,011, 19,506, 20,124 und 20,568 umfasst;
und wobei
das amorphe Perindopril durch ein Verfahren umfassend die folgenden schritte erhalten wird:
(i) die sekundäre Aminogruppe der stereospezifischen Aminosäure N'-[(s)-carbethoxy-1-butyl]-(S)-alanin der Formel II: 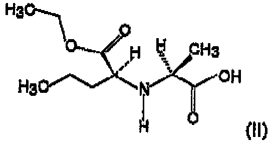 wird mit Trimethylsilylgruppen geschützt, um die Verbindung der Formel III zu erhalten: 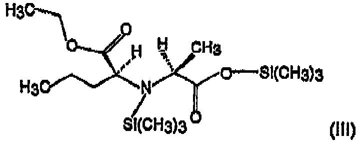
ii) Verbindung (III) wird dann durcrch Reaktion mit einem aktivierenden Agens, ausgewählt aus der Gruppe bestehend aus Thionylchlorid und dem Thionylchlorid-Komplex mit 1-H-Benzotriazol, aktiviert, um das Säurechlorid der Formel IV zu erhalten: 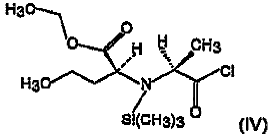
(iii) das Säurechlorid der Formel IV wird reagiert mit der (2S, 3aS, 7aS)-Octayhdroindolo-2-Carbonsäure, die eine geschützte Carboxylgruppe der Formel V besitzt: 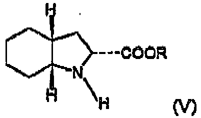 wobei R eine Trimethylsilylgruppe ist, um ein geschütztes Perindopril-Derivat der Formel VI zu erhalten: 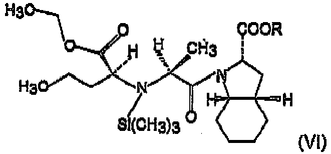
(iv) das Derivat der Formel VT wird dann zu Perindopril der Formel I durch Entfernen der Trimethylsilylgruppen während des Schritts des Entfernens von Verunreinigungen durch Extraktion mit einer wässrigen Lösung umgewandelt,
(v) die resultierende organische Phase enthaltend Perindopril wird durch Evaporation des Lösungsmittels in Vakuum getrocknet,
(vi) das rohe Perindopril wird in Methylenchlorid aufgelöst und durch eine Kieselgelschicht gefiltert,
(vii) die Fraktion, die Perindopril enthält, wird unter einem starken Vakuum evaporiert;
oder
das amorphe Perindopril durch das Verfahren umfassend die folgenden Schritte erhalten wird:
(i) die sekundäre Aminogruppe der stereospezifischen Aminosäure N-[(S)-Carbethoxy-1-butyl]-(S)-alanin der Formel II: 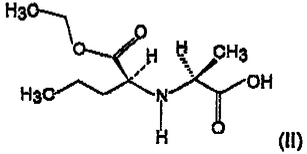 wird mit Trimethylsilylgruppen geschützt, um die Verbindung der Formel III zu erhalten: 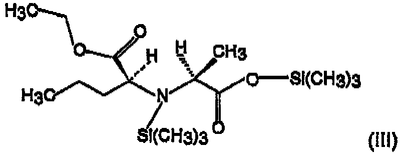
(ii) Verbindung (III) wird dann durch Reaktion mit einem aktivierenden Agens, ausgewählt aus der Gruppe bestehend aus Thionylchlorid und dem Thionylchlorid-Komplex mit 1-H-Benzotriazol, aktiviert, um das Säurechlorid der Formel IV zu erhalten: 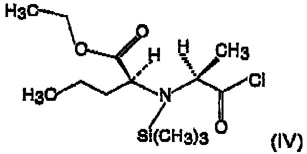
(iii) das Säurechlorid der Formel IV wird reagiert mit der (2S, 3aS, 7aS)-Octahydroindolo-2-Carbonsäure, die eine geschützte Carboxylgruppe der Formel V besitzt: 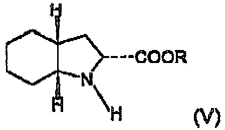 wobei R eine
Benzylgruppe ist, um ein geschütztes Perindopril-Derivat der Formel VI zu erhalten: 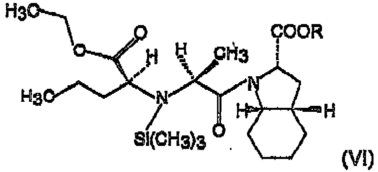
(iv) Verunreinigen der finalen Reaktionsmischung werden durch Extraktion mit einer wässrigen Lösung entfernt,
(v) die organische Phase wird durch eine Aluminumoxidschicht gefiltert,
(vi) die Fraktion, die das Derivat der Formel VI enthält, wird unter Vakuum evaporiert,
(vii) das Derivat der Formel VI wird dann zu Perindopril der Formel I durch Entfernen aller Schutzgruppen durch katalytische Hydrogenierung umgewandelt,
(viii) der Katalysator wird durch Filtration entfernt,
(ix) das Lösungsmittel wird unter einem Vakuum evaporiert.

2. Verfahren gemäß Anspruch 1, wobei die neue kristalline Form durch das folgende Röntgenpulverdiffraktionadiagramm gekennzeichnet ist, gemessen unter Verwerdung eines Diffraktometers (Cuk_{α}-Antikathode), ausgedrückt in Form von Einheiten des Winkels 2θ, mit dem Zwischenschicht-Abstand d und der entsprechenden Intensität oder relativen Intensität:
| Winkel 2θ | Zwischenschicht-Abstand dx10⁻¹⁰m | Impuls x sec⁻¹ | relative Intensität % |
|---|---|---|---|
| 3,656 | 24,15027 | 72,0 | 2,4 |
| 5,101 | 17,31096 | 35,0 | 1,2 |
| 6,378 | 13,84687 | 3042 | 100 |
| 6,884 | 12,83052 | 165 | 5,4 |
| 7,362 | 11,99804 | 154 | 5,1 |
| 7,842 | 11,26490 | 67,0 | 2,2 |
| 8,615 | 10,25496 | 434 | 14,3 |
| 9,373 | 9,42781 | 444 | 14,6 |
| 9,719 | 9,09325 | 141 | 4,6 |
| 11,090 | 7,97163 | 612 | 20,1 |
| 11,403 | 7,75386 | 514 | 16,9 |
| 12,240 | 7,22540 | 403 | 13,2 |
| 12,524 | 7,06220 | 567 | 18,6 |
| 12,840 | 6,88883 | 354 | 11,6 |
| 13,294 | 6,65438 | 271 | 8,9 |
| 13,800 | 6,41169 | 400 | 13,1 |
| 14,082 | 6,28392 | 636 | 20,9 |
| 14,572 | 6,07381 | 477 | 15,7 |
| 15,230 | 5,81266 | 848 | 27,9 |
| 15,990 | 5,53829 | 700 | 23,0 |
| 17,215 | 5,14680 | 1403 | 46,1 |
| 17,958 | 4,93545 | 764 | 25,1 |
| 18,640 | 4,75633 | 328 | 10,8 |
| 19,011 | 4,66440 | 772 | 25,4 |
| 19,506 | 4,54719 | 891 | 29,3 |
| 20,124 | 4,40874 | 643 | 21,1 |
| 20,568 | 4,31453 | 850 | 27,9 |
| 21,572 | 4,11599 | 466 | 15,3 |
| 22,025 | 4,03235 | 472 | 15,5 |
| 22,983 | 3,86650 | 492 | 16,2 |
| 23,933 | 3,71499 | 607 | 20,0 |
| 23,347 | 3,65278 | 422 | 13,9 |
| 25,321 | 3,51446 | 328 | 10,8 |
| 25,520 | 3,48752 | 319 | 10,5 |
| 26,105 | 3,41063 | 327 | 10,7 |
| 29,885 | 3,31348 | 447 | 14,7 |
| 27,612 | 3,22783 | 266 | 8,7 |
| 28,560 | 3,12283 | 268 | 8,8 |
| 28,756 | 3,10197 | 309 | 10,2 |
| 29,428 | 3,03266 | 267 | 8,8 |
| 30,303 | 2,94702 | 277 | 9,1 |
| 30,593 | 2,91983 | 280 | 9,2 |
| 31,413 | 2,84537 | 261 | 8,6 |

3. Verfahren zur Herstellung der neuen kristallinen Form von Perindopril gemäß Anspruch 1 oder Anspruch 2, wobei der niedrige Dialkylether Diethylether ist.

## Revendications

1. Procédé de préparation de la nouvelle forme cristalline du périndopril de formule I : 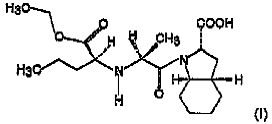 comprenant les étapes de dissolution de périndopril amorphe dans un éther dialkylique inférieur contenant jusqu'à 8 atomes de carbone, et de cristallisation de ladite nouvelle forme cristalline du perindopril à partir de la solution résultante, dans lequel ladite nouvelle forme cristalline est **caractérisée par** un spectre de diffraction de poudre des rayons X comprenant les pics caractéristiques à (2θ) : 5,101, 8,615, 9,373, 11,090, 11,403, 15,230, 15,990, 17,215, 19,011, 19,506, 20,124 et 20,568 ;
et dans lequel
ledit périndopril amorphe est obtenu par le procédé comprenant les étapes suivantes :
(i) le groupe amino secondaire de l'acide aminé stéréospécifique N-[(S)-carbéthoxy-1-butyl]-(S)-alanine de formule II : 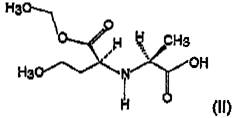 est protégé par des groupes triméthylsilyle afin de donner le composé de formule III : 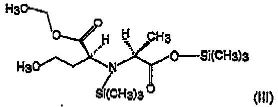
(ii) le composé (III) est ensuite active par réaction avec un agent d'activation choisi dans le groupe constitué du chlorure de thionyle et du complexe de chlorure de thionyle avec du 1-H-benzotriazole pour donner le chlorure d'acide de formule IV : 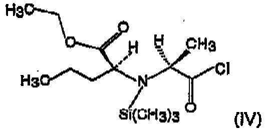
(iii) le chlorure d'acide de formule IV est mis à réagir avec de l'acide (2S, 3as, 7aS)-octahydroindolo-2-carboxylique portant un groupe carboxylique protégé de formule V : 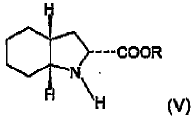 dans laquelle R est un groupe triméthylsilyle, pour donner un dérivé de périndopril protégé de formule VI : 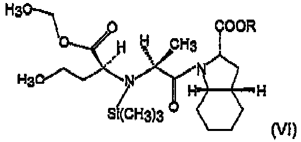
(iv) le dérivé de formule VI est ensuite converti en périndopril de formule I par élimination des groupes triméthylsilyle pendant l'étape d'élimination des impuretés par extraction avec une solution aqueuse,
(v) la phase organique résultante contenant le périndopril est séchée par évaporation du solvant sous vide,
(vi) le périndopril brut est dissous dans du chlorure de méthylène et filtré à travers une couche de gel de silice,
(vii) la fraction contenant le périndopril est évaporée sous un vide poussé ou
ledit périndopril amorphe est obtenu par le procédé comprenant les étapes suivantes :
(i) le groupe amino secondaire de l'acide aminé stéréospécifique N-[(S)-caxbéthoxy-1-butyl]-(S)-alanine de formule II : 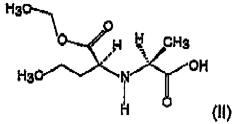 est protégé par des groupes triméthylsilyle afin de donner le composé de formule III : 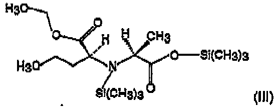
(ii) le composé (III) est ensuite active par réaction avec un agent d'activation choisi dans le groupe constitué du chlorure de thionyle et du complexe de chlorure de thionyle avec du 1-H-benzotriazole pour donner le chlorure d'acide de formule IV : 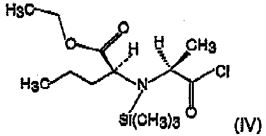
(iii) le chlorure d'acide de formule IV est mis à réagir avec de l'acide (2S, 3aS, 7a5)-octahydroindolo-2-carboxylique portant un groupe carboxylique protège de formule V : 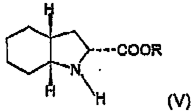 dans laquelle R est un groupe benzylique, pour donner un dérivé de périndopril protégé de formule VI : 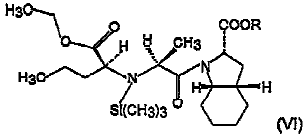
(iv) les impuretés du mélange réactionnel final sont éliminées par extraction avec une solution aqueuse,
(v) la phase organique est filtrée sur une couche d'oxyde d'aluminium,
(vi) la fraction contenant le dérivé de formule VI est évaporée sous vide,
(vii) le dérivé de formule VI est ensuite converti en périndopril de formule I par élimination de tous les groupes protecteurs par hydrogénation catalytique,
(viii) le catalyseur est éliminé par filtration,
(ix) le solvant est évaporé sous vide.

2. Procédé selon la revendication 1, dans lequel ladite nouvelle forme cristalline est **caractérisée par** le diagramme de diffraction des rayons X de poudre suivant, mesuré à l'aide d'un diffractomètre (anticathode K_{α} du Cu), exprimé en termes d'unités d'angle 2θ, avec la distance interplanaire d et l'intensité ou l'intensité relative Correspondante :
| Angle 2θ | Distance interplanaire d x 10⁻¹⁰ m | impulsions d'intensité, s⁻¹ | Intensité relative, % |
|---|---|---|---|
| 3,656 | 24,15027 | 72,0 | 2,4 |
| 5,101 | 17,31096 | 35,0 | 1,2 |
| 6,378 | 13,84687 | 3042 | 100 |
| 6,884 | 12,83052 | 165 | 5,4 |
| 7,362 | 11,99804 | 154 | 5,1 |
| 7,842 | 11,26490 | 67,0 | 2,2 |
| 8,615 | 10,25496 | 434 | 14,3 |
| 9,373 | 9,42781 | 444 | 14,6 |
| 9,719 | 9,09325 | 141 | 4,6 |
| 11,090 | 7,97163 | 612 | 20,1 |
| 11,403 | 7,75386 | 514 | 16,9 |
| 12,240 | 7,22540 | 403 | 13,2 |
| 12,524 | 7,06220 | 567 | 18,6 |
| 12,840 | 6,88883 | 354 | 11,6 |
| 13,294 | 6,65438 | 271 | 8,9 |
| 13,000 | 6,41169 | 400 | 13,1 |
| 14,082 | 6,28392 | 636 | 20,9 |
| 14,572 | 6,07381 | 477 | 15,7 |
| 15,230 | 5,81266 | 848 | 27,9 |
| 15,990 | 5,53829 | 700 | 23,0 |
| 17,215 | 5,14680 | 1403 | 46,1 |
| 17,958 | 4,93545 | 764 | 25,1 |
| 18,640 | 4,75633 | 328 | 10,8 |
| 19,011 | 4,66440 | 772 | 25,4 |
| 19,506 | 4,54719 | 891 | 29,3 |
| 20,124 | 4,40874 | 643 | 21,1 |
| 20,568 | 4,31453 | 850 | 27,9 |
| 21,572 | 4,11599 | 466 | 15,3 |
| 22,025 | 4,03235 | 472 | 15,5 |
| 22,983 | 3,86650 | 492 | 16,2 |
| 23,933 | 3,71499 | 607 | 20,0 |
| 23,347 | 3,65278 | 422 | 13,9 |
| 25,321 | 3,51446 | 328 | 10,8 |
| 25,520 | 3,48752 | 319 | 10,5 |
| 26,105 | 3,41063 | 327 | 10,7 |
| 29,885 | 3,31348 | 447 | 14,7 |
| 27,612 | 3,22783 | 266 | 8,7 |
| 28,560 | 3,12283 | 268 | 8,8 |
| 28,756 | 3,10197 | 309 | 10,2 |
| 29,428 | 3,03266 | 267 | 8,8 |
| 30,303 | 2,94702 | 277 | 9,1 |
| 30,593 | 2,91983 | 280 | 9,2 |
| 31,413 | 2,84537 | 261 | 8,6 |

3. Procédé de préparation de la nouvelle forme cristalline du périndopril selon la revendication 1 ou la revendication 2, dans lequel ledit éther dialkylique inférieur est l'éther diéthylique.
